(19) Europäisches Patentamt
European Patent Office
Office européen des brevets

(11) EP 1 581 486 B1

(12) EUROPEAN PATENT SPECIFICATION

(45) Date of publication and mention of the grant of the patent:
**29.07.2009 Bulletin 2009/31**

(21) Application number: **03779081.3**

(22) Date of filing: **18.09.2003**

(51) Int Cl.:
***C12Q 1/48*** *(2006.01)*

(86) International application number:
**PCT/US2003/029702**

(87) International publication number:
**WO 2004/026820 (01.04.2004 Gazette 2004/14)**

(54) **METHOD FOR ASSAYING COMPOUNDS THAT DECREASE THE ACTIVITY OF POLY(ADP-RIBOSE)-POLYMERASE (PARP)**

VERFAHREN ZUM TESTEN VON VERBINDUNGEN, DIE DIE WIRKUNG VON POLY(ADP-RIBOSE)-POLYMERASE (PARP) VERMINDERN

PROCEDE POUR DOSER DES COMPOSES QUI DIMINUENT L'ACTIVITE D'UNE POLY(ADP-RIBOSE)-POLYMERASE (PARP)

(84) Designated Contracting States:
**AT BE BG CH CY CZ DE DK EE ES FI FR GB GR HU IE IT LI LU MC NL PT RO SE SI SK TR**

(30) Priority: **19.09.2002 US 412136 P**
**24.01.2003 GB 0301628**

(43) Date of publication of application:
**05.10.2005 Bulletin 2005/40**

(73) Proprietor: **Aventis Pharmaceuticals Inc.**
**Bridgewater, New Jersey 08807 (US)**

(72) Inventors:
- **LI, Zhuyin, Julie**
  **Flemington, NJ 08822 (US)**
- **MEHDI, Shujaath**
  **Manville, NJ 08835 (US)**

(74) Representative: **Bouvet, Philippe et al**
**Aventis Pharma S.A.**
**Direction des Brevets Tri LEO/144**
**20 Avenue Raymond Aron**
**92165 Antony Cedex (FR)**

(56) References cited:
**EP-A- 1 227 322** **WO-A2-02/059352**

- **ARMSTRONG S ET AL: "Application of a Fluorometric Assay for Characterization of the Catalytic Competency of a Domain III Fragment of Pseudomonas aeruginosa Exotoxin A" April 2001 (2001-04), ANALYTICAL BIOCHEMISTRY, ACADEMIC PRESS, NEW YORK, NY, US, PAGE (S) 26-33 , XP002988535 ISSN: 0003-2697 * page 26 - page 28 ***
- **MOHAMMED SARWAR NASIR ET AL: "Fluorescence Polarization: an analytical tool for immunoassay and drug discovery" COMBINATORIAL CHEMISTRY AND HIGH THROUGHPUT SCREENING, HILVERSUM, NL, vol. 2, no. 4, 1999, pages 177-190, XP001062258 ISSN: 1386-2073**
- **LEFEVRE C ET AL: "Texas Res-X and rhodamine Red-X, new derivatives of sulforhodamine 101 and lissamine rhodamine B with improved labeling and fluorescence properties." BIOCONJUGATE CHEMISTRY. 1996 JUL-AUG, vol. 7, no. 4, July 1996 (1996-07), pages 482-489, XP002410467 ISSN: 1043-1802**
- **DECKER P. ET AL: 'An Improved Nonisotopic Test to Screen a Large Series of New Inhibitor Molecules of Poly(ADP-ribose) Polymerase Activity for Therapeutic Applications' CLINICAL CANCER RESEARCH vol. 5, May 1999, pages 1169 - 1172, XP002324565**
- **ARMSTRONG S. ET AL: 'Application of a Fluorometric Assay for Characterization of the Catalytic Competency of a Domain III Fragment of Pseudomonas aeruginosa Exotoxin A' ANALYTICAL BIOCHEMISTRY vol. 292, April 2001, pages 26 - 33, XP002988535**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **COROMINAS M. ET AL: 'Poly(ADP-ribosylation) at Successive Stages of Rooster Spermatogenesis' THE JOURNAL OF BIOLOGICAL CHEMISTRY vol. 260, no. 30, 25 December 1985, pages 16269 - 16273, XP002988536**

## Description

FIELD OF THE INVENTION

The present invention relates to a novel and useful method for determining whether a compound or agent can decrease or inhibit the activity of PARP.

BACKGROUND OF THE INVENTION

Ischemia is an extremely deleterious conditions, and it is related to a wide variety of cardiovascular problems, such as stroke, heart attack, hypertension, etc. Consequently, a treatment of ischemia may ameliorate the problems associated with cardiovascular disease, and provide a patient suffering from such a disease an increased quality of life. Although the mechanism of ischemia is not clear, it has been discovered to be related to the activity of proteins in the body. One such protein is an enzyme called poly(ADP-ribose) polymerase (PARP). PARP is a nuclear enzyme that is involved in DNA repair. More particularly, upon its activation by damaged DNA, PARP catalyzes the transfer of an ADP ribose unit from $NAD^+$ to a glutamate of a protein acceptor, e.g. a histone. PARP then adds additional ADP ribose units to the substrate forming an ADP-ribose chain on the substrate. However, it is believed that the activity of PARP depletes cellular $NAD^+$, and thus energy currency, e.g. ATP. As a result, cell death and ischemia result. Moreover, ischemia inherently produces extensive DNA damage by free radicals, which in turn causes further activation of PARP. As a result, a cycle of degradation is formed which leads to substantial damage to the subject. Consequently, a compound or agent that is found to decrease or inhibit PARP may readily have applications in treating ischemia or other cardiovascular diseases related to ischemia, such as stroke, heart attack, etc.

Screening for potent enzyme inhibitors from large compound libraries is one of the most popular approaches for identifying compounds or agents that may eventually be sold in the market place as drugs. However, conventional screening formats can impose extreme demands on limiting resources, which include individual compound supplies, purified enzyme production and human resources. Therefore, development of miniaturizable assays has been pursued to alleviate this problem However, heretofore known methods for performing such an assay inherently possess shortcomings.

For example, conventional filtration based methods, scintillation assays, and ELISA type such as described in EP 1.227.322 assays involve numerous washing steps, radioactive isotopes (which are expensive to dispose of), expensive reagents, multiple wash steps, high assay variation, or a combination of these shortcomings. Moreover, such heretofore known methods are very difficult to apply to miniaturized ultra high throughput screening programs.

The article of Armstrong S et al (April 2001 (2001-04), Analytical Biochemistry, Academic Press, New York, NY, US, page(s) 26-33) relates to the exotoxin A (ETA) from *Pseudomonas aeruginosa*. It teaches a fluorometric assay in which the enzyme domain of ETA catalyses the transfer of ADP-ribose from NAD+ to the diphthamide residue in eukaryotic translation factor eEF-2.

The application WO 02/059352 discloses in very general terms a method for the detection of enzyme-catalyzed cleavage and linking reactions. More than 24 enzymes are listed, among them the transferases, and more especially the DNA or RNA polymerases.

The article of Lefevre C et al (BIOCONJUGATE CHEMISTRY. 1996 JUL-AUG, vol.7, no.4, July 1996 (1996-07), pages 482-489) discloses many examples of use of Texas Red-X, but none relates to the use in an enzyme reaction.

The article of Mohammed Sarwar Nasir et al: (COMBINATORIAL CHEMISTRY AND HIGH THROUGHPUT SCREENING, HILVERSUM, NL, vol.2, no.4, 1999, pages 177-190) teaches in general terms the use of Fluorescence polarization (FP).

The only enzymes mentioned (part B), are proteases, DNAses, RNAses and $\alpha$-amylase actions.

Accordingly, what is needed is a new and useful method for identifying compounds or agents that modulate, and particularly decrease or inhibit the activity of PARP.

What is also needed is a method for determining whether a compound or agent decreases or inhibits PARP, wherein the method does not require the use of numerous washing steps or dangerous radioactive isotopes to perform the method.

What is also needed is a method for identifying a compound or agent that modulates, and particularly decreases or inhibits the activity of PARP, wherein the method can be performed in a high throughput fashion. Such a method would permit one of ordinary skill in the art to assay many compounds quickly and inexpensively.

The citation of any reference herein should not be construed as an admission that such reference is available as "Prior Art" to the instant application.

SUMMARY OF THE INVENTION

There is provided, in accordance with a present invention, a new and useful method of evaluating compounds or agents for their ability to decrease or inhibit the activity of PARP that does not utilize radioactive isotopes, does not require numerous washing steps, and can be performed *in vitro, in vivo*, in a cell based manner, or in an isolated manner. Moreover, a method of the present invention can readily be performed in a high throughput manner.

Broadly, the present invention extends to a method for determining whether a compound or agent decreases the activity of a poly(ADP-ribose)-polymerase (PARP). In such a method a mixture comprising PARP, the compound or agent to be assayed and a substrate reagent solution is incubated. The substrate solution comprises $NAD^+$, $NAD^+$ having an ADP ribose group labeled with a fluorescence label, protein such as histone that can act as the acceptor substrate, and DNA as a cofactor. The DNA may be endogenous DNA found in the medium in which a method of the present invention is being performed, or alternatively added in order to activate the PARP. After a period of incubation, the mixture and a control mixture (described *infra*) are illuminated with plane polarized light having a wavelength at which the fluorescence label fluoresces. The fluorescence polarization of the mixture, as well as the control mixture are measured and compared. A fluorescence polarization measurement of the mixture that is less than a fluorescence polarization measurement of the control mixture indicates the compound or agent decreases or inhibits the activity of PARP.

Furthermore, the present invention extends to a method for determining whether a compound or agent decreases or inhibits the activity of PARP, as described herein, wherein the incubating step of such a method has a duration of at least about ten (10) minutes. More particularly, the duration of the incubating step can range from about 10 minutes to at least 2, 3, or even 4 hours.

Numerous fluorescence labels have applications in a method of the present invention. Particular examples include, but certainly are not limited to phycoerythrin (PE), Texas red (TR), rhodamine, a free lanthanide series salt, a chelated lanthanide series salt, BODIPY (Molecular Probes), ALEXA (Molecular Probes), or CyDye (Amersham Biotech). Naturally, the wavelength of plane polarized light utilized, as well as the wavelength of the fluorescent emission, will vary depending upon the fluorescence label used. In a particular example, the fluorescence label utilized is Texas red (TR). Hence, the wavelength of the plane polarized light is 590 nm, and the wavelength of the emission is 620 nm.

In addition, in a method of the present invention, $NAD^+$ having the ADP ribose group labeled with a fluorescence label may comprise a linker molecule to which the ADP ribose group and the fluorescence label are bound. Examples of linker molecules having applications herein include, but certainly are not limited to aminobutyric acid, aminocaproic acid, 7-aminoheptanoic acid, 8-aminocaprylic acid, Fmoc-aminocaproic acid, one or more β-alanines, an isothiocyanate group, a succinimidyl ester, a sulfonal halide, a carbodiimide, and a $C_6$ spacer arm: $-CH_2-CH_2-CH_2-CH_2-CH_2-CH_2-$. In a particular embodiment, the fluorescence label is Texas Red, and the linker that links the ADP ribose group and the fluorescence label is a $C_6$ spacer arm.

Moreover, the present invention extends to a method for determining whether a compound or agent decreases or inhibits the activity of a poly(ADP-ribose)-polymerase (PARP), wherein the method comprises the steps of:

(a) Incubating a mixture for at least 10 minutes, wherein the mixture comprises:

(i) PARP enzyme activated by DNA;
(ii) the compound or agent; and
(iii) a substrate reagent solution comprising $NAD^+$, $NAD^+$ having an ADP ribose group labeled with a fluorescence label, DNA, and histone;

(b) illuminating the mixture of step (a) and a control mixture with plane polarized light having a wavelength at which the fluorescence label fluoresces, and measuring the fluorescence polarization of the mixture of step (a) and the control mixture; and
(c) comparing the measurements of step (b).

A fluorescence polarization measurement of the mixture having a value that is less than the value of the fluorescence polarization measurement of control mixture indicates the compound or agent decreases or inhibits the activity of the PARP enzyme.

Moreover, the present application extends to a method for determining whether a compound or agent decreases or inhibits the activity of a poly(ADP-ribose)-polymerase (PARP), wherein the method comprises the steps of:

(a) incubating a mixture that comprises:

(i) activated PARP enzyme;

(ii) the compound or agent; and

(iii) a substrate reagent solution comprising $NAD^+$, $NAD^+$ having an ADP ribose group labeled with Texas Red, DNA, and histone;

(b) illuminating the mixture of step (a) and a control mixture with plane polarized light having a wavelength of 590 nm, and measuring the fluorescence polarization of the mixture of step (a) and the control mixture at a wavelength of 620 nm; and

(c) comparing the measurements of step (b).

A fluorescence polarization measurement of the mixture having a value less than the value of the fluorescence polarization measurement of the control mixture indicates the compound or agent decreases the activity of the PARP enzyme.

In addition, the present application extends to a method for determining whether a compound or agent decreases the activity of a poly(ADP-ribose)-polymerase (PARP) comprising the steps of:

(a) incubating a mixture for at least about 10 minutes, wherein the mixture comprises:

(i) activated PARP enzyme;

(ii) the compound or agent; and

(iii) a substrate reagent solution comprising $NAD^+$, $NAD^+$ having an ADP ribose group labeled with Texas Red, DNA and histone;

(b) illuminating the mixture of step (a) and a control mixture with plane polarized light having a wavelength of 590 nm, and measuring the fluorescence polarization of the mixture of step (a) and the control mixture at a wavelength of 620 nm; and

(c) comparing the measurements of step (b).

A fluorescence polarization measurement of the mixture is less than the fluorescence polarization measurement of control mixture indicates the compound or agent decreases the activity of the PARP enzyme.

Accordingly, it is an aspect of the present invention to provide a useful and heretofore unknown method for evaluating the ability of a compound or agent to decrease or inhibit the activity of a PARP enzyme. Such a compound or agent may have applications in treating ischemia, stroke or another type of cardiovascular disease in a subject.

It is another aspect of the present invention to provide a method for evaluating the ability of a compound or agent to decrease or inhibit the activity of a PARP enzyme, wherein such a method does not require laborious and time consuming washing steps, the use of radioactive isotopes, or the use of expensive reagents.

It is still another aspect of the present invention to provide a method for evaluating the ability of a compound or agent to decrease or inhibit the activity of a PARP enzyme that can be performed *in vivo, in vitro*, cell based, or in an isolated fashion.

It is yet still another aspect of the present invention to provide a method for evaluating the ability of a compound or agent to decrease or inhibit the activity of a PARP enzyme that can be performed in a high throughput manner.

These and other aspects of the present invention will be better appreciated by reference to the following drawings and Detailed Description.

## BRIEF DESCRIPTION OF THE DRAWINGS

FIG.1 is a schematical view of the chemical reaction catalyzed by PARP. An ADP ribose group is cleaved from $NAD^+$ and then bound to a glutamate residue of a protein acceptor substrate. In a method of the present invention, the protein acceptor substrate is a histone. PARP then catalyzes the binding of additional ADP ribose groups to the ADP ribose group bound to glutamate, to produce a chain.

FIG. 2: Simulated effect of fluorescence lifetime on fluorescence polarization (FP) as a function of molecular mass.

FIG. 3: Graph of an inhibition curve using isoquinoline-1,5-diol which is a known inhibitor of PARP. This data, generated with a method of the present invention, demonstrates a method of the present invention can determine whether a compound or agent decreases the activity of PARP.

FIG. 4 is the chemical structure of an $NAD^+$ having an ADP ribose group labeled with a fluorescence label.

FIG. 5: Schematical view of the principle of the FP Implementation in a method of the present invention.

FIG. 6: Amino acid sequence of PARP enzyme used in the Example (SEQ ID NO:1).

## DETAILED DESCRIPTION OF THE INVENTION

The present invention is based upon the discovery that surprisingly and unexpectedly, fluorescence polarization can be utilized to identify compounds or agents that decrease or inhibit the activity of a PARP enzyme. As a result, sufficient energy levels, e.g., ATP will be present in the cell, and ischemia, stroke or other types of cardiovascular disease may be successfully treated. The reaction that PARP catalyzes is schematically shown in FIG. 1.

Broadly, the present invention extends to a method for determining whether a compound or agent decreases the activity of a poly(ADP-ribose)-polymerase (PARP) comprising the steps of:

(a) incubating a mixture comprising:

(i) PARP enzyme activated by DNA;
(ii) the compound or agent; and
(iii) a substrate reagent solution comprising $NAD^+$, $NAD^+$ having an ADP ribose group labeled with a fluorescence label, DNA and histone;

(b) illuminating the mixture of step (a) and a control mixture with plane polarized light having a wavelength at which the fluorescence label fluoresces, and measuring the fluorescence polarization of the mixture of step (a) and the control mixture; and
(c) comparing the measurements of step (b),

wherein the fluorescence polarization measurement of the mixture that is less than the fluorescence polarization measurement of control mixture indicates the compound or agent decreases the activity of the PARP enzyme.

Numerous terms and phrases used throughout the instant Specification and appended Claims are defined below. Accordingly:

As used herein, the terms "compound" or "agent" refer to any composition presently known or subsequently discovered. Examples of compounds or agents having applications herein include organic compounds (e.g., man made, naturally occurring and optically active), peptides (man made, naturally occurring, and optically active, i.e., either D or L amino acids), carbohydrates, nucleic acid molecules, etc.

As used herein, the term "enzyme" refers to a biomolecule, such as a protein or RNA, that catalyzes a specific chemical reaction. It does not affect the equilibrium of the catalyzed reaction. Rather, the enzyme enhances the rate of reaction by lowering the energy of activation.

As used herein, the term "cofactor" refers to a substance such as an inorganic ion, a coenzyme, a nucleic acid molecule, etc. that is required for enzyme activity. In a particular embodiment, the cofactor is DNA. The DNA may be naturally occurring, i.e., endogenous, if a method of the present invention is being performed in a cell based manner. Alternatively, the DNA may be added to the mixture in order to activate the PARP.

As used herein, the term "activated PARP" refers to PARP that is in the presence of the cofactor DNA. As explained above, the DNA may be endogenously present within a cell or the lysate of a cell. If a method of the present invention is being performed under conditions in which DNA is not naturally present, DNA may be added in order to activate the PARP.

As used herein, the term "substrate" refers to the composition upon which an enzyme acts. The substrate can be either a "donor substrate", which is the name of the specie upon which the enzyme catalyzes the cleavage of a particular moiety, or the "acceptor substrate," which is the specie to which the enzyme catalyzes the binding of the moiety. In a particular embodiment, $NAD^+$ is the donor substrate, a histone is the acceptor substrate, and the moiety is an ADP ribose group of $NAD^+$. PARP catalyzes the transfer of the ADP ribose group from the $NAD^+$ to a glutamate residue of the histone.

As used herein, the term "fluorescence label" refers to chemical that fluoresces when illuminated with a particular wavelength of light, wherein the compound is bound directly to a compound of interest, or alternatively, is bound to a linker that is in turn bound to the compound of interest. Examples of fluorescence labels having applications in a method of the present invention include, but certainly are not limited to phycoerythrin (PE), Texas red (TR), rhodamine, a free lanthanide series salt, a chelated lanthanide series salt, BODIPY, ALEXA, CyDye, etc. A particular fluorescence label having applications in a method of the present invention is Texas red.

As used herein, the term "tracer" refers to a detectably labeled moiety that is added to the mixture containing the compound or agent to be assayed. Chemically, but for the label, it is the same as the moiety that is transferred from the donor substrate to the receptor substrate. As a result, the tracer and the moiety cleaved from the donor substrate compete to bind with the acceptor substrate. This competition and its impact on fluorescence polarization measurements are described *infra*. In a particular embodiment, the "tracer" is $NAD^+$ having an ADP ribose group with a fluorescence label, such as Texas Red. The fluorescence label may be bonded directly to the ADP ribose group of $NAD^+$, or alternatively,

both the ADP ribose group and the fluorescence label may be bound to a linker.

As used herein, the terms "linker" and "linker molecule" may be used interchangeably, and refer to a chemical moiety to which the fluorescence label and the compound of interest, e.g., the ADP ribose group of $NAD^+$, are bound. Particular examples of linkers having applications in the present invention include aminobutyric acid, aminocaproic acid, 7-aminoheptanoic acid, 8-aminocaprylic acid, Fmoc-aminocaproic, one or more β-alanines, an isothiocyanate group, a succinimidyl ester, a sulfonal halide, a $C_6$ spacer arm, or a carbodiimide, to name only a few. A particular example of a linker having applications in the present invention is a $C_6$ spacer arm.

As used herein, the term "control mixture" refers to a mixture containing the same reagents, cells, etc. in the same amounts as the mixture containing the compound or agent being assayed, and is treated in the same manner as the mixture containing the compound or agent being assayed, except the control mixture does not contain the compound or agent.

<u>Fluorescence Polarization</u>

Fluorescence polarization is a technique that is used to study interactions among molecules. The principles behind this technique are dependent upon the size of molecules being evaluated. In particular, when a fluorescent molecule is illuminated with plane polarized light at a particular wavelength, electrons at their ground state in the molecule are promoted to an excited state. After approximately 4-5 nanoseconds, these excited electrons decay back to their ground state. It is during this decay that the molecule emits a fluorescence signal. In fluorescence polarization, this fluorescence emission can be detected only if the molecule remains stationary throughout the excited state. If the molecule moves or rotates during the excited state, the fluorescence emission will be in a different plane of light than that of the polarized light that excited the electrons of the fluorescent molecule. As a result, a fluorescence emission will not be detected. It is well accepted that the smaller the molecule, the greater its mobility and rotation. Hence, a small molecule will produce a substantially smaller FP signal than a larger molecule, which will remain relatively stationary during the excitation period. It is this property of molecules that fluorescence polarization utilizes. In a fluorescence polarization assay with respect to a ligand and its receptor, a tracer, i.e., the ligand labeled with a fluorescence label, and the receptor to which the ligand binds, are placed in solution. The ligand and the tracer then compete with each other to bind to the receptor. The solution is then illuminated with plane polarized light, and a signal is then detected. If not much unlabeled ligand present in the solution, then the majority of receptors present will bind to the tracer. Since the receptor is a large molecule (relative to the ligand), and consequently, rotates very slowly in solution, a signal will be obtained from the fluorescence of the label. In contrast, if there is a large amount of unlabeled ligand present, then a majority of receptors will bind with the ligand, which rotates more quickly than the receptor. As a result, a fluorescence signal produced by the tracer, if produced at all, will be substantially smaller than the previously obtained signal produced by the tracer bound to the receptor. It is the difference between these signals that enable one of ordinary skill in the art to determine whether the ligand is present, and its concentration. In the present invention, the ligand is an ADP ribose group cleaved from $NAD^+$, and the receptor is a histone. The PARP enzyme, the compound or agent being assayed, $NAD^+$ and a tracer comprising $NAD^+$ having an ADP ribose group with a fluorescence label, are mixed together. If the compound or agent being evaluated decreases or inhibits the activity of PARP, then the majority of fluorescent labeled ADP ribose groups will remain bound to $NAD^+$, which is a much smaller molecule (on a relative scale) than histone. However, in the control mixture, the enzyme is free to remove labeled ADP ribose groups from $NAD^+$ and to catalyze the binding of these groups to histone. Thus, the control mixture will have a relatively large measured FP value. Consequently, if the compound or agent decreases the activity of PARP, the measured FP value for the mixture will be less than the measured FP value for the control. Hence, a method of the present invention readily permits one of ordinary skill in the art to determine whether a particular compound or agent being evaluated decreases the activity of PARP.

Fluorescence polarization is measured in millipolarization units, or mP. It is calculated by the equation:

$$P = \frac{I_v - I_h}{I_v + I_h} \qquad \text{(I)}$$

where $I_v$ and $I_h$ are the fluorescence vertical and horizontal intensities, respectively. Fluorescence lifetime and molecular rotational correlation time are the prime factors in determining fluorescence polarization, as described in Perrin's equation:

$$\left[\frac{1}{\overline{P}} - \frac{1}{3}\right] = \left[\frac{1}{\overline{P}_o} - \frac{1}{3}\right] * \left[1 + \frac{\tau_f}{\tau_c}\right] \qquad \text{(II)}$$

where $\overline{P}$ is the steady state polarization, $\overline{P}_0$ is the initial limiting polarization of the fluorophore, $\tau_f$ is fluorescence lifetime, and $\tau_c$ is molecular rotational correlation time. For spherical molecules, $\tau_c$ is related to hydrated molecular volume ($V_h$) as:

$$\tau_c = \frac{V_h \eta}{kT} \quad \text{(III)}$$

where $\eta$ is the viscosity of the environment and T is temperature. FIG. 2 shows simulated effects of fluorescence lifetime on fluorescence polarization as a function of spherical molecular weight. As indicated by the simulation, by using a short lifetime fluorescence tracer, such as fluorescein or Rhodamine (Texas Red) to label a small donor substrate, the transfer of the chemical moiety with the tracer to another mass of greater size can be monitored through the fluorescence polarization changes. In the absence of molecular transfer, the fluorescence tracer can rotate quickly, hence the emission is depolarized. Upon transfer of the tracer to a large molecule, the fluorescence tracer remains relatively stationary during the lifetime of the fluorophore, therefore the emitted light will have a high degree of polarization (the Scheme set forth in FIG. 5). For non-spherical molecules, the FP value may deviate from the predicted value in Figure 1, however, the relative relationships between FP, $\tau_f$ and $V_h$ hold.

Conditions

As explained above, a method of the present invention can be performed *in vivo, in vitro*, or in an isolated form, wherein all the reagents, enzymes, substrates, etc. were previously isolated and maintained in a buffer solution, such as TRIS, TRIS HCl, HEPEs, or phosphate buffer under physiological conditions (i.e., physiological pH, temperature, etc.), or in a cell-based manner. In a particular example, the conditions for a method of the present invention may comprise 4.0 nM of Texas Red labeled $NAD^+$, 100 $\mu$M unlabeled $NAD^+$, 0.025 mg/ml Histone, 1 mM DTT, 0.1 mg/ml DNA, 50 mM TRIS, pH 8.0, 5 mM $MgCl_2$, 10 $\mu$M test compound, 7% Glycerol, enzyme concentration batch dependent (0.5 to 2 $\mu$g /mL). Total reaction volume: 6.5 $\mu$l. Reaction temperature: 22 °C in humidified chamber. Reaction time: 2 hours. However, these conditions are only an example of conditions under which to perform a method of the present invention. One of ordinary skill in the art using routine laboratory techniques and knowledge can readily modify such conditions and successfully perform a method of the present invention.

Search of Libraries for Candidate Compounds or Agents that Decrease or Inhibit the Activity of PARP

Conventionally, new chemical entities with useful properties are generated by identifying a chemical compound (called a "lead compound") with some desirable property or activity, creating variants of the lead compound, and evaluating the property and activity of those variant compounds. However, the current trend is to shorten the time scale for all aspects of drug discovery. Because of the ability to test large numbers quickly and efficiently, high throughput screening (HTS) methods are replacing conventional lead compound identification methods.

In a particular embodiment, high throughput screening methods involve providing a library containing a large number of potential therapeutic compounds (candidate compounds). Such "combinatorial chemical libraries" are then screened with a method of the present invention to identify those library members (particular chemical species or subclasses) that display a desired characteristic activity. The compounds thus identified can serve as conventional "lead compounds" or can themselves be used as potential or actual therapeutics.

Combinatorial chemical libraries

Combinatorial chemical libraries are a preferred means to assist in the generation of new chemical compound leads. A combinatorial chemical library is a collection of diverse chemical compounds generated by either chemical synthesis or biological synthesis by combining a number of chemical "building blocks" such as reagents. For example, a linear combinatorial chemical library such as a polypeptide library is formed by combining a set of chemical building blocks called amino acids in every possible way for a given compound length (i.e., the number of amino acids in a polypeptide compound). Millions of chemical compounds can be synthesized through such combinatorial mixing of chemical building blocks. For example, one commentator has observed that the systematic, combinatorial mixing of 100 interchangeable chemical building blocks results in the theoretical synthesis of 100 million tetrameric compounds or 10 billion pentameric compounds (Gallop *et al.* (1994) 37(9): 12331250).

Preparation of combinatorial chemical libraries is well known to those of ordinary skill in the art. Such combinatorial chemical libraries include, but are not limited to peptide libraries (*see, e.g.*, U.S. Patent 5,010,175, Furka (1991) Int. J. Pept. Prot. Res., 37: 487-493, Houghton et al. (1991) Nature, 354: 84-88). Peptide synthesis is by no means the

only approach envisioned and intended for use with the present invention. Other chemistries for generating chemical diversity libraries can also be used. Such chemistries include, but certainly are not limited to: peptoids (PCT Publication No WO 91/19735, 26 Dec. 1991), encoded peptides (PCT Publication WO 93/20242, 14 Oct. 1993), random biooligomers (PCT Publication WO 92/00091, 9 Jan. 1992), benzodiazepines (U.S. Pat. No. 5,288,514), diversomers such as hydantoins, benzodiazepines and dipeptides (Hobbs et al., (1993) Proc. Nat. Acad. Sci. USA 90: 69096913), vinylogous polypeptides (Hagihara et al. (1992) J. Amer. Chem. Soc. 114: 6568), nonpeptidal peptidomimetics with a Beta D Glucose scaffolding (Hirschmann et al., (1992) J. Amer. Chem. Soc. 114: 92179218), analogous organic syntheses of small compound libraries (Chen et al. (1994) J. Amer. Chem. Soc. 116: 2661), oligocarbamates (Cho, et al., (1993) Science 261:1303), and/or peptidyl phosphonates (Campbell et al., (1994) J. Org. Chem. 59: 658). *See, generally*, Gordon et al., (1994) J. Med. Chem. 37:1385, nucleic acid libraries, peptide nucleic acid libraries (*see, e.g.,* U.S. Patent 5,539,083) antibody libraries (*see, e.g.,* Vaughn et al. (1996) Nature Biotechnology, 14(3): 309-314), and PCT/US96/10287), carbohydrate libraries (*see, e.g.*, Liang et al. (1996) Science, 274: 1520-1522, and U.S. Patent 5,593,853), and small organic molecule libraries (*see, e.g.,* benzodiazepines, Baum (1993) C&EN, Jan 18, page 33, isoprenoids U.S. Patent 5,569,588, thiazolidinones and metathiazanones U.S. Patent 5,549,974, pyrrolidines U.S. Patents 5,525,735 and 5,519,134, morpholino compounds U.S. Patent 5,506,337, benzodiazepines 5,288,514, and the like).

Devices for the preparation of combinatorial libraries are commercially available (*see*, *e.g.*, 357 MPS, 390 MPS, Advanced Chem Tech, Louisville KY, Symphony, Rainin, Woburn, MA, 433A Applied Biosystems, Foster City, CA, 9050 Plus, Millipore, Bedford, MA).

A number of well known robotic systems have also been developed for solution phase chemistries. These systems include automated workstations like the automated synthesis apparatus developed by Takeda Chemical Industries, LTD. (Osaka, Japan) and many robotic systems utilizing robotic arms (Zymate II, Zymark Corporation, Hopkinton, Mass.; Orca, HewlettPackard, Palo Alto, Calif.) which mimic the manual synthetic operations performed by a chemist. Any of the above devices are suitable for use with the present invention. The nature and implementation of modifications to these devices (if any) so that they can operate as discussed herein will be apparent to persons skilled in the relevant art. In addition, numerous combinatorial libraries are themselves commercially available (*see, e.g.*, ComGenex, Princeton, N.J.; Asinex, Moscow, Ru; Tripos, Inc., St. Louis, MO; ChemStar, Ltd, Moscow, RU; 3D Pharmaceuticals, Exton, PA; Martek Biosciences, Columbia, MD, *etc*.).

## High throughput assays of chemical libraries

Naturally, a method of the present invention, which employs fluorescence polarization, is readily amenable to high throughput screening. High throughput screening systems are commercially available *(see, e.g.,* Zymark Corp., Hopkinton, MA; Air Technical Industries, Mentor, OH; Beckman Instruments, Inc. Fullerton, CA; Precision Systems, Inc., Natick, MA, *etc*.). These systems typically automate entire procedures including all sample and reagent pipetting, liquid dispensing, timed incubations, and final readings of the microplate in detector(s) appropriate for the assay. These configurable systems provide high throughput and rapid start up as well as a high degree of flexibility and customization. The manufacturers of such systems provide detailed protocols for the various high throughput. Thus, for example, Zymark Corp. provides technical bulletins describing screening systems for detecting the modulation of gene transcription, ligand binding, and the like.

The present invention may be better understood by reference to the following non-limiting Example, which is provided as exemplary of the invention. The following Example is presented in order to more fully illustrate the particular embodiments of the present invention. It should in no way be construed, however, as limiting the broad scope of the invention.

## EXAMPLE

Provided herein is a novel and useful method for determining whether a compound or agent decreases or inhibits the activity of Poly(ADP-Ribose)-Polymerase enzyme (PARP). PARP is a nuclear enzyme normally involved in DNA repair. Upon activation by damaged DNA, PARP transfers an ADP ribose unit from $NAD^+$ to a glutamate of a protein acceptor, and subsequently adds more ADP-ribose units to the first ADP-ribose to cause elongation of the ADP-ribose chain. Furthermore, PARP has been implicated to be involved in cell death in ischemia or neurotoxic insult by depleting cellular $NAD^+$ and thus energy currency ATP. Consequently, a compound or agent that decreases or inhibits the activity of PARP may well have applications in treating such conditions.

In a particular embodiment of the present invention described herein, a fluorescence tracer, Texas Red, is covalently linked to $NAD^+$. The chemical structure of this tracer is set forth in FIG. 4.

The transfer of Texas Red-ADP ribose to protein substrates (histone and PARP itself) can be monitored through the rotational speed of the fluorescence tracer by the fluorescence polarization method. The assay measures the ability of the test compounds to inhibit the enzyme to transfer fluorescence labeled ADP to protein substrates.

Materials

The materials used in this Example, as well as the vendors from which they were obtained, and their catalog numbers are set forth in the table below:

| Materials | *Supplier* | *Catalog Number* | *MW* | *Function* |
|---|---|---|---|---|
| 10 ul tips | JENOPTIK/MATRIX | 5550 | | |
| 1536-well black plates | MATRIX/Greiner | 4515A | | |
| plate cover | RAININ | 96-LID-60S | | |
| | | | | |
| cold NAD+ | Sigma | N-6522 | 663.4 | substrate |
| TR-NAD+ | LJL | custom | 1520.5 | tracer/substrate |
| DTT | Sigma | D-5542 | 154.2 | antioxidant |
| Histone | Sigma | H-9250 | | substrate |
| DNA, sonicated | Sigma | D-1501 | | cofactor |
| 6(5H)-phenanthridinone | Aldrich | 29,963-4 | 195.2 | stand inhibitor (IC50 =0.2uM) |
| TRIS buffer, pH 8.0 | Sigma | T-4753 | (20 Liters) | buffer |
| MgCl2.6H2O | Sigma | M-2670 | 203.3 | cofactor |
| Glycerol | Sigma | G-6279 | 92.09 | reduce evaporation |
| Tween-80 | Aldrich | 27,436-4 | | reduce non-specific absorptio |
| | | | | |
| YobiWell 384/1536 | Jenoptik/Matrix | | | liquid handling |
| LJL Acquest | LJL Bioscience | | | detector |

Reagents used:

A PARP *baculovirus* which encodes the amino acid sequence of FIG. 6 (SEQ ID NO:1), was used to infect sf (9) cells. After PARP was expressed, it was partially purified by 40-70% ammonia sulfate cuts, and then re-suspended in TRIS buffer containing 25% (v/v) glycerol.

Preparation of the $NAD^+$ tracer having an ADP ribose group with a fluorescence label

In this example of a method of the present invention, the tracer utilizes Texas Red as the fluorescence label. However, as explained above, numerous fluorescence labels have applications in a method of the present invention. However, the wavelength of plane polarized light used to cause the label to fluoresce must have a wavelength that is known to cause the particular label to fluoresce. Moreover, as explained above, the fluorescence label and the ADP ribose group may be bound directly to each, or instead, a linker can be used to which they both are bound. The chemical structure of the tracer used in this Example is set forth in FIG. 4. The method for producing the tracer is set forth below:

Texas Red succinimidyl ester (Molecular Probes) was added to a solution of the NAD+ (Sigma Chemicals) in dry methylene chloride. The reaction was stirred under nitrogen in the dark for 24 hours. Purification was performed by reverse phase HPLC chromatography using a water/acetonitrile gradient with 0.05% TEA as a modifier.

Other Reagents

Other reagents used are set forth in the table below:

| Reagnets | Chemicals | Solvent | Compound Conc. | |
|---|---|---|---|---|
| | *all stocks should be kept at -80˚C* | | Conc. | Unit |
| *Reaction buffer* | TRIS, hemisodium<br>pH 8.0<br>MgCl2.6H2O | H2O | 50<br><br>5 | mM<br><br>mM |
| *Control inhibitor* | 6(5H)-Phenathridinone (prepare 10 mM stock dissolved in DMSO) | Reaction buffer | 65 | uM |
| *Substrate reagent* | TR-$NAD^+$<br>(Prepare 500 nM stock dissolved in 0.1% Tween-80)<br>Glycerol<br>$NAD^+$ (20 mM stock)<br>Histone (10 mg/ml stock) | Reaction buffer | 8.7<br><br>7<br>216.7<br>0.05 | nM<br><br>%<br>uM<br>mg/ml |
| *Enzyme reagent* | DTT (10 mM stock)<br>DNA, sonicated (1 mg/ml stock)<br>Enzyme | Reaction buffer | 2.2<br>0.22<br>batch depend | mM<br>mg/ml<br> |

Conditions:

4.0 nM of Texas Red labeled $NAD^+$, 100 μM unlabeled $NAD^+$, 0.025 mg/ml Histone, 1 mM DTT, 0.1 mg/ml DNA, 50 mM TRIS, pH 8.0, 5 mM $MgCl_2$, 10 μM of the compound or agent, 7% Glycerol, enzyme concentration batch dependent. Total reaction volume: 6.5 μl. Reaction temperature: 22 ˚C in humidified chamber. Reaction time: 2 hours.

Procedure:

The method of the present invention was performed manually in 1536 well black plates. However, as explained above, a method of the present invention can readily be automated and performed in a high throughput manner.

In this method, to 3.0 μl substrate reagent was added 0.5 μl of 130 μM compound or control compound, as described *infra*. Then, 3.0 μl enzyme reagent and 3.0 μl of reaction buffer were added to the well. This mixture was then permitted to incubate for about 2 hours at room temperature. Naturally, a control mixture was prepared that is identical except that the control mixture does not contain the compound or agent.

After incubation, this mixture and the control mixture were illuminated with plane polarized light having a wavelength of 590 nm, and the fluorescence polarization of the mixture and the control mixture were measured using a fluorescence filter set with an excitation wavelength of 590 nm, and an emission wavelength of 620 nm. The measurements were made with a fluorescence polarization plate reader in fluorescence polarization (FP) mode. These two measurements were then compared to determine whether the fluorescence polarization measurement of the mixture containing the compound or agent is less than that of the control. Such a finding would show that the enzyme in the control mixture functioned correctly while the enzyme in the mixture containing the compound or agent being assayed exhibited decreased activity. As a result, the compound or agent being assayed decreased PARP activity, and thus may have applications in treating cardiovascular disease, ischemia, stroke, etc.

Evaluation of Method with Control compound

To confirm that a method of the present invention functions correctly, it was performed using 1,5-isoquinolinediol, a known inhibitor of PARP as the compound or agent, i.e., as a control compound. It has an $IC_{50}$ of 379 nm. The structure of this control is set forth below:

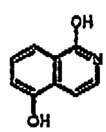

The procedure described above was performed using isoquinoline-1,5-diol purchased from Sigma Chemicals (Saint Louse, MO, catalog number I-138). The results of these experiments are set forth in the inhibition curve set forth

in PIG. 3. The inhibition curve of this figure graphically shows that a method of the present invention can readily identify a compound or agent that decreases or inhibits the activity of PARP.

Conclusion

The data obtained using a known inhibitor of PARP in a method of the present invention clearly demonstrates that the present invention provides an easy, quick and accurate way of evaluating compounds for their ability to decrease PARP activity, and yet does not require multiple washings or radioactive isotopes. Moreover, a method of the present invention can readily be used in a high throughput manner for the evaluation of thousands of compounds or agents in a very short period of time.

The present invention is not to be limited in scope by the specific embodiments described herein. Indeed, various modifications of the invention in addition to those described herein will become apparent to those skilled in the art from the foregoing description and the accompanying figures. Such modifications are intended to fall within the scope of the appended claims.

It is further to be understood that all base sizes or amino acid sizes, and all molecular weight or molecular mass values, given for nucleic acids or polypeptides are approximate, and are provided for description.

## Claims

**1.** A method for determining whether a compound or agent decreases the activity of a poly(ADP-ribose)-polymerase (PARP) comprising the steps of:

(a) incubating a mixture comprising:

(i) PARP enzyme activated by DNA;
(ii) the compound or agent; and
(iii) a substrate reagent solution that comprises $NAD^+$, $NAD^+$ having an ADP ribose group labeled with a fluorescence label DNA, and histone;

(b) illuminating the mixture of step (a) and a control mixture with plane polarized light having a wavelength at which the fluorescence label fluoresces, and measuring the fluorescence polarization of the mixture of step (a) and the control mixture; and
(c) comparing the measurements of step (b),

wherein the fluorescence polarization measurement of the mixture having a value that is less than the fluorescence polarization measurement of control mixture indicates the compound or agent decreases the activity of the PARP enzyme.

**2.** The method of Claim 1, wherein the incubating step (a) has a duration of at least 10 minutes.

**3.** The method of Claim 2, wherein the incubating step has a duration ranging from 10 minutes to at least 2 hours.

**4.** The methods of Claim 1, wherein the fluorescence label comprises phycoerythrin (PE), Texas red (TR), rhodamine, a free lanthanide series salt, a chelated lanthanide series salt, BODIPY, ALEXA, or CyDye.

**5.** The method of Claim 4, wherein the fluorescence label is Texas red (TR).

**6.** The method of Claim 5, wherein the wavelength of the plane polarized light is 590 nm.

**7.** The method of Claim 5, wherein the $NAD^+$ having an ADP ribose group labeled with Texas Red comprises a linker molecule to which the ADP ribose group and the Texas Red are bound.

**8.** The method of Claim 7, wherein the linker molecule is selected from the group consisting of aminobutyric acid, aminocaproic acid, 7-aminoheptanoic acid, 8-aminocaprylic acid, Fmoc-aminocaproic acid, one or more β-alanines, an isothiocyanate group, a succinimidyl ester, a sulfonal halide, a carbodiimide, and a $C_6$ spacer.

**9.** The method of Claim 8, wherein the linker is the $C_6$ spacer.

## Patentansprüche

1. Verfahren zur Bestimmung davon, ob eine Verbindung oder ein Agens die Aktivität einer Poly-(ADP-Ribose)-Polymerase (PARP) vermindert, wobei das Verfahren die folgenden Schritte umfasst:

    (a) Inkubieren eines Gemischs, umfassend:

        (i) PARP-Enzym, aktiviert durch DNA,
        (ii) die Verbindung bzw. das Agens und
        (iii) eine Substratreagenslösung, die $NAD^+$, $NAD^+$ mit einer mit einer Fluoreszenzmarkierung markierten ADP-Ribose-Gruppe, DNA und Histon enthält;

    (b) Bestrahlen des Gemischs aus Schritt (a) und eines Kontrollgemischs mit linear polarisiertem Licht mit einer Wellenlänge, bei der die Fluoreszenzmarkierung fluoresziert, und Messen der Fluoreszenzpolarisation des Gemischs aus Schritt (a) sowie des Kontrollgemischs; und
    (c) Vergleichen der Messungen aus Schritt (b), wobei ein Wert für die Fluoreszenzpolarisationsmessung des Gemischs, der unterhalb der Fluoreszenzpolarisationsmessung des Kontrollgemischs liegt, anzeigt, dass die Verbindung bzw. das Agens die Aktivität des PARP-Enzyms vermindert.

2. Verfahren nach Anspruch 1, wobei der Inkubationsschritt (a) eine Dauer von wenigstens 10 Minuten aufweist.

3. Verfahren nach Anspruch 2, wobei der Inkubationsschritt eine Dauer im Bereich von 10 Minuten bis mindestens 2 Stunden aufweist.

4. Verfahren nach Anspruch 1, wobei die Fluoreszenzmarkierung Phycoerythrin (PE), Texas-Rot (TR), Rhodamin, ein freies Salz der Lanthanoidenreihe, ein chelatiertes Salz der Lanthanoidenreihe, BODIPY, ALEXA oder CyDye umfasst.

5. Verfahren nach Anspruch 4, wobei es sich bei der Fluoreszenzmarkierung um Texas-Rot (TR) handelt.

6. Verfahren nach Anspruch 5, wobei die Wellenlänge des linear polarisierten Lichts 590 nm beträgt.

7. Verfahren nach Anspruch 5, wobei das $NAD^+$ mit einer mit Texas-Rot markierten ADP-Ribose-Gruppe ein Linker-Molekül umfasst, an das die ADP-Ribose-Gruppe und das Texas-Rot gebunden sind.

8. Verfahren nach Anspruch 7, wobei das Linker-Molekül aus der Gruppe, bestehend aus Aminobuttersäure, Aminocapronsäure, 7-Aminoheptansäure, 8-Aminocaprylsäure, Fmoc-Aminocapronsäure, einem oder mehreren β-Alaninen, einer Isothiocyanat-Gruppe, einem Succinimidylester, einem Sulfonalhalogenid, einem Carbodiimid und einem $C_6$-Spacer, ausgewählt ist.

9. Verfahren nach Anspruch 8, wobei es sich bei dem Linker um den $C_6$-Spacer handelt.

## Revendications

1. Procédé pour déterminer si un composé ou un agent diminue l'activité d'une poly(ADP-ribose) polymérase (PARP), comprenant les étapes consistant à :

    (a) incuber un mélange comprenant :

        (i) une enzyme PARP activée par l'ADN ;
        (ii) le composé ou l'agent ; et
        (iii) une solution de réactif substrat qui comprend du $NAD^+$, le $NAD^+$ ayant un groupe ADP ribose marqué avec un marqueur fluorescent, de l'ADN, et une histone ;

    (b) éclairer le mélange de l'étape (a) et un mélange témoin avec une lumière polarisée dans un plan, ayant une longueur d'onde à laquelle le marqueur fluorescent émet une fluorescence ; et mesurer la polarisation de fluorescence du mélange de l'étape (a) et du mélange témoin ; et

(c) comparer les mesures de l'étape (b),

dans lequel la mesure de la polarisation de fluorescence du mélange, ayant une valeur qui est inférieure à celle de la mesure de la polarisation de fluorescence du mélange témoin, indique que le composé ou l'agent diminue l'activité de l'enzyme PARP.

**2.** Procédé selon la revendication 1, dans lequel l'étape (a) d'incubation a une durée d'au moins 10 minutes.

**3.** Procédé selon la revendication 2, dans lequel l'étape d'incubation a une durée allant de 10 minutes à au moins 2 heures.

**4.** Procédé selon la revendication 1, dans lequel le marqueur fluorescent comprend la phycoérythrine (PE), le rouge de Texas (TR), la rhodamine, un sel de la série des lanthanides libres, un sel de la série des lanthanides chélatés, le BODIPY, l'ALEXA ou le colorant Cy.

**5.** Procédé selon la revendication 4, dans lequel le marqueur fluorescent est le rouge de Texas (TR).

**6.** Procédé selon la revendication 5, dans lequel la longueur de la lumière polarisée dans un plan est de 590 nm.

**7.** Procédé selon la revendication 5, dans lequel le $NAD^+$ ayant un groupe ADP ribose marqué avec du rouge de Texas comprend une molécule lieuse à laquelle le groupe ADP ribose et le rouge de Texas sont liés.

**8.** Procédé selon la revendication 7, dans lequel la molécule lieuse est choisie dans le groupe constitué par l'acide aminobutyrique, l'acide aminocaproïque, l'acide 7-aminoheptanoïque, l'acide 8-aminocaprylique, l'acide Fmoc-aminocaproïque, une ou plusieurs β-alanines, un groupe isothiocyanate, un ester de succinimidyle, une halogénure sulfonique, un carbodiimide, et un espaceur en $C_6$.

**9.** Procédé selon la revendication 8, dans lequel le lieur est l'espaceur en $C_6$.

FIGURE 1

## The Reaction Catalyzed by Poly(ADP-Ribose) Polymerase

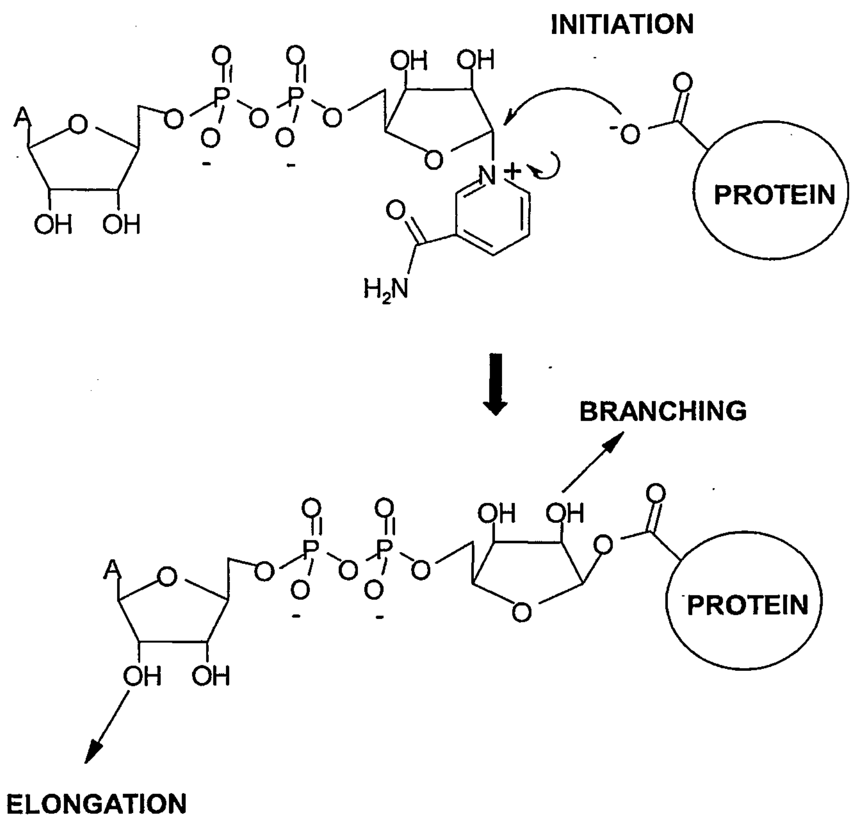

FIGURE 2

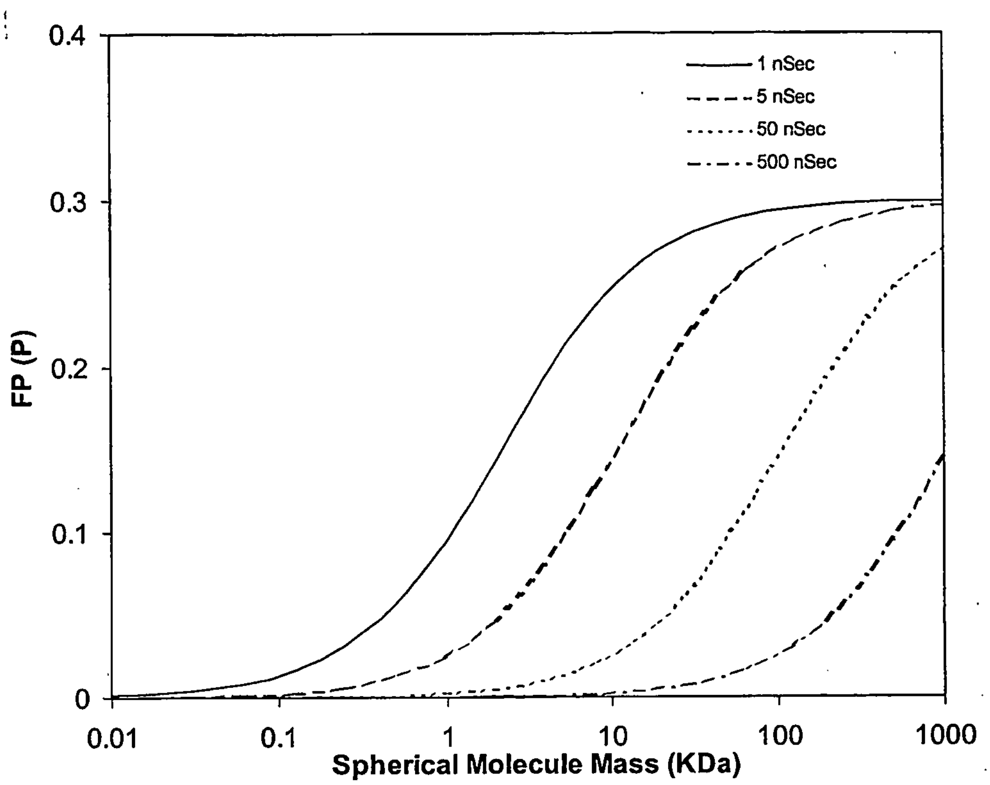
0.4
0.3
0.2
0.1
0
FP (P)
1 nSec
5 nSec
50 nSec
500 nSec
0.01
0.1
1
10
100
1000
Spherical Molecule Mass (KDa)

FIGURE 3

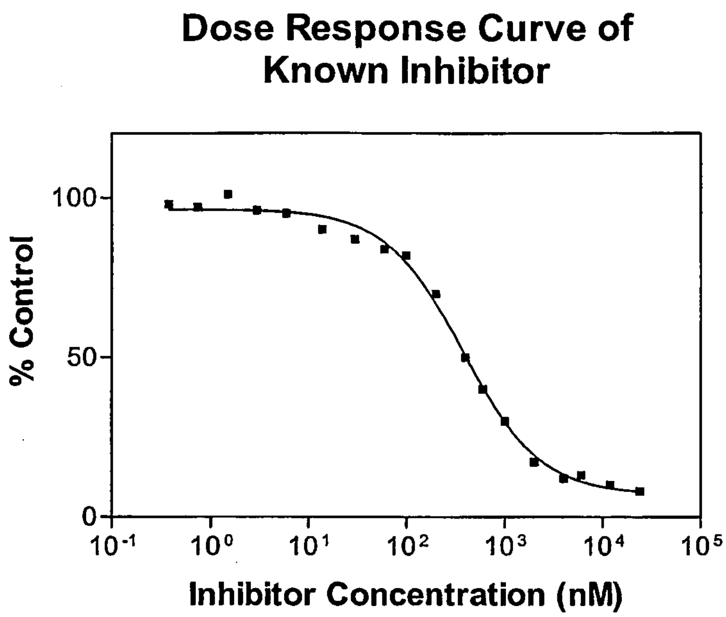
Dose Response Curve of Known Inhibitor
% Control
100
50
0
10⁻¹
10⁰
10¹
10²
10³
10⁴
10⁵
Inhibitor Concentration (nM)

FIGURE 4

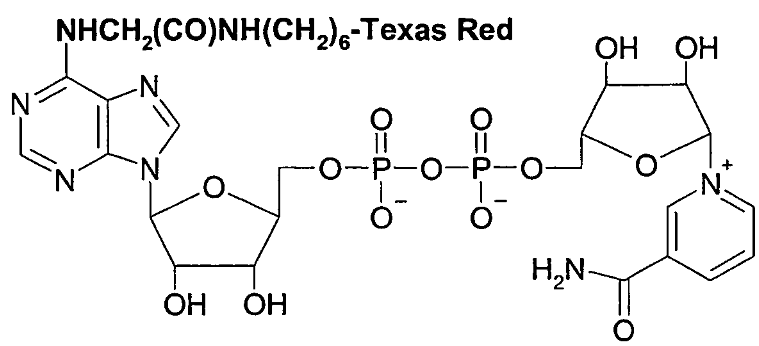

FIGURE 5

*Principle of the FP Implementation of the PARP Assay*

A)

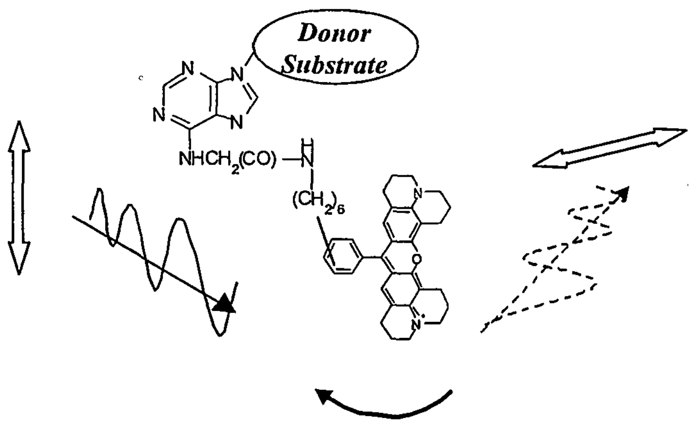


B)

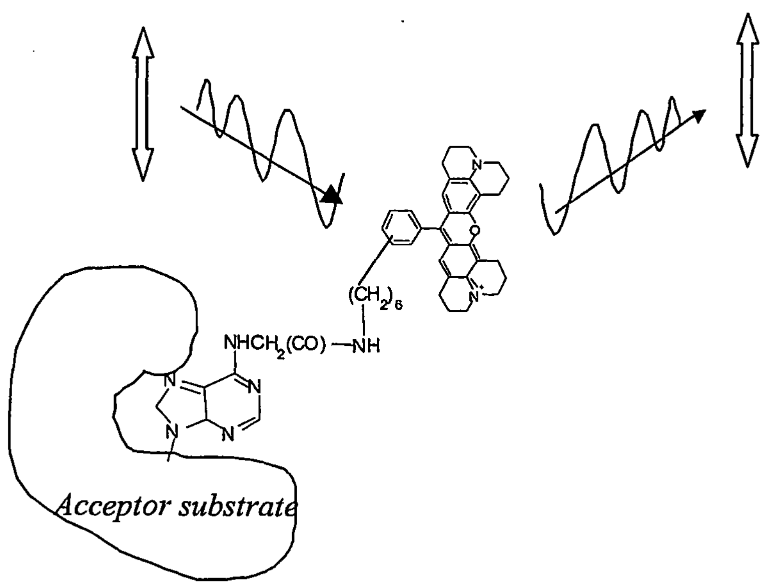

FIGURE 6

MSYYHHHHHHDYDIPTTENLYFQGAMGSMAESSDKLYRVEYAKSGRASCKKCSESIPKDSLRMAIMV
QSPMFDGKVPHWYHFSCFWKVGHSIRHPDVQVDGFSELRWDDQQKVKKTAEAGGVTGKGQDGIGSK
AEKTLGDFAAEYAKSNRSTCKGCMEKIEKGQVRLSKKMVDPEKPQLGMIDRWYHPGCFVKNREELGF
RPEYSASQLKGFSLLATEDKEALKKQLPGVKSEGKRKGDEVDGVDEVAKKKSKKEKDKDSKLEKALK
AQNDLIWNIKDELKKVCSTNDLKELLIFNKQQVPSGESAILDRVADGMVFGALLPCEECSGQLVFKSDA
YYCTGDVTAWTKCMVKTQTPNRKEWVTPKEFREISYLKKLKVKKQDRIFPPETSASVAATPPPSTASAP
AAVNSSASADKPLSNMKILTLGKLSRNKDEVKAMIEKLGGKLTGTANKASLCISTKKEVEKMNKKMEE
VKEANIRVVSEDFLQDVSASTKSLQELFLAHILSPWGAEVKAEPVEVVAPRGKSGAALSKKSKGQVKEE
GINKSEKRMKLTLKGGAAVDPDSGLEHSAHVLEKGGKVFSATLGLVDIVKGTNSYYKLQLLEDDKENR
YWIFRSWGRVGTVIGSNKLEQMPSKEDAIEHFMKLYEEKTGNAWHSKNFTKYPKKFYPLEIDYGQDEE
AVKKLTVNPGTKSKLPKPVQDLIKMIFDVESMKKAMVEYEIDLQKMPLGKLSKRQIQAAYSILSEVQQA
VSQGSSDSQILDLSNRFYTLIPHDFGMKKPPLLNNADSVQAKVEMLDNLLDIEVAYSLLRGGSDDSSKD
PIDVNYEKLKTDIKVVDRDSEEAEIIRKYVKNTHATTHNAYDLEVIDIFKIEREGECQRYKPFKQLHNRR
LLWHGSRTTNFAGILSQGLRIAPPEAPVTGYMFGKGIYFADMVSKSANYCHTSQGDPIGLILLGEVALGN
MYELKHASHISKLPKGKHSVKGLGKTTPDPSANISLDGVDVPLGTGISSGVNDTSLLYNEYIVYDIAQVN
LKYLLKLKFNFKTS
LW

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- EP 1227322 A
- WO 02059352 A
- US 5010175 A
- WO 9119735 A
- WO 9320242 A
- WO 9200091 A
- US 5288514 A
- US 5539083 A
- US 9610287 W
- US 5593853 A
- US 5569588 A
- US 5549974 A
- US 5525735 A
- US 5519134 A
- US 5506337 A

### Non-patent literature cited in the description

- **Armstrong S et al.** Analytical Biochemistry. Academic Press, April 2001, 26-33
- **Lefevre C et al.** *BIOCONJUGATE CHEMISTRY,* July 1996, vol. 7 (4), 482-489
- **Mohammed Sarwar Nasir et al.** *COMBINATORIAL CHEMISTRY AND HIGH THROUGHPUT SCREENING,* 1999, vol. 2 (4), 177-190
- **Furka.** *Int. J. Pept. Prot. Res.,* 1991, vol. 37, 487-493
- **Houghton et al.** *Nature,* 1991, vol. 354, 84-88
- **Hobbs et al.** *Proc. Nat. Acad. Sci. USA,* 1993, vol. 90, 69096913
- **Hagihara et al.** *J. Amer. Chem. Soc.,* 1992, vol. 114, 6568
- **Hirschmann et al.** *J. Amer. Chem. Soc.,* 1992, vol. 114, 92179218
- **Chen et al.** *J. Amer. Chem. Soc. 1,* 1994, vol. 16, 2661
- **Cho et al.** *Science,* 1993, vol. 261, 1303
- **Campbell et al.** *J. Org. Chem.,* 1994, vol. 59, 658
- **Gordon et al.** *J. Med. Chem.,* 1994, vol. 37, 1385
- **Vaughn et al.** *Nature Biotechnology,* 1996, vol. 14 (3), 309-314
- **Liang et al.** *Science,* 1996, vol. 274, 1520-1522
- **Zymate II.** Zymark Corporation, Hopkinton, Mass. Orca, HewlettPackard
- **ComGenex ; Princeton, N.J.** Asinex, Moscow, Ru. Tripos, Inc,
- **Moscow, RU.** 3D Pharmaceuticals, Exton, PA. Martek Biosciences
- **Zymark Corp. ; Hopkinton, MA ; Mentor, OH.** Air Technical Industries. Beckman Instruments, Inc,